# EUROPÄISCHE PATENTSCHRIFT

(11) **EP 1 035 127 B1**
(45) Veröffentlichungstag und Bekanntmachung des Hinweises auf die Patenterteilung: **08.10.2003**
(21) Anmeldenummer: 00105011.1
(22) Anmeldetag: 09.03.2000
(51) Int. Cl.: C07F 9/6512

(54) **Verfahren zur Herstellung von N-[5-(Diphenylphosphinoylmethyl)-4-(4-fluorphenyl)-6-isopropylpyrimidin-2-yl]-N-methylmethansulfonamid**
Process for the preparation of N-[5-(Diphenylphosphinoylmethyl)-4-(4-fluorphenyl)-6-isopropylpyrimidin-2-yl]-N-methylmethansulfonamide
Procédé pour la préparation de la N-[5-(Diphenylphosphinoylmethyl)-4-(4-fluorphenyl)-6-isopropylpyrimidin-2-yl]-N-methylmethansulfonamide

(30) Priorität: 10.03.1999 EP 99104785; 10.03.1999 EP 99104786
(43) Veröffentlichungstag der Anmeldung: 13.09.2000
(73) Patentinhaber: Lonza AG, 4052 Basel (CH)
(72) Erfinder: Brieden, Walter, Dr., 3902 Brig-Glis (CH); Veith, Ulrich, Dr., 3930 Visp (CH)
(74) Vertreter: Winter, Brandl, Fürniss, Hübner, Röss, Kaiser, Polte Partnerschaft

(56) Entgegenhaltungen:
- EP-A- 0 521 471
- BUSS A.D. ET AL.: "The stereocontrolled Horner-Wittig reaction: synthesis of disubstituted alkenes" JOURNAL OF THE CHEMICAL SOCIETY, PERKIN TRANSACTIONS 1.,1985, Seiten 2307-2325, XP000907385 CHEMICAL SOCIETY. LETCHWORTH., GB ISSN: 0300-922X
- HALMANN M: "Solvolysis of chlorodialkyl- and diarylphosphines, dialkyl phosphorochloridites and dialkyl phosphinyl chlorides" PHOSPHORUS SULFUR RELAT. ELEM. (PREEDF,0308664X);1988; VOL.40 (3-4); PP.251-61, XP000913585 Weizmann Inst. Sci.;Isot. Dep.; Rehovot; 76100; Israel (IL)
- WATANABE M ET AL: "Synthesis and biological activity of methanesulfonamide pyrimidine- and N-methanesulfonyl pyrrole-substituted 3,5-dihydroxy-6-heptenoates, a novel series of HMG-CoA reductase inhibitors" BIOORG. MED. CHEM. (BMECEP,09680896);1997; VOL.5 (2); PP.437-444, XP000911018 Shionogi and Company, Ltd.;Shionogi Res. Lab.; Osaka; 553; Japan (JP)

## Beschreibung

Die vorliegende Erfindung betrifft ein Verfahren zur Herstellung von N-[5-(Diphenylphosphinoylmethyl)-4-(4-fluorphenyl)-6-isopropylpyrimidin-2-yl]-N-methyhnethansulfonamid der Formel

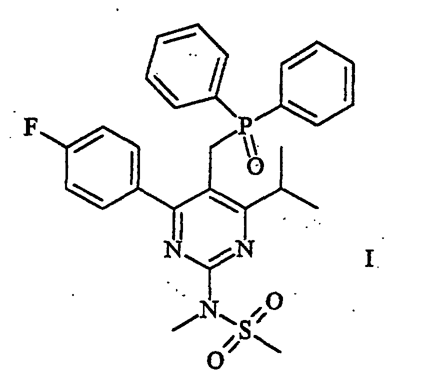

Das erfindungsgemäss herstellbare N-[5-(Diphenylphosphinoylmethyl)-4-(4-fluorphenyl)-6-isopropylpyrimidin-2-yl]-N-methylmethansulfonamid (I) ist ein Zwischenprodukt zur Herstellung pharmazeutischer Wirkstoffe, beispielsweise von HMG-Co A Reduktase Inhibitoren (*Bioorg. Med. Chem.* **1997**, *5*, 437).

Die Aufgabe der Erfindung bestand darin, einen verbesserten Zugang zum o.g. Zwischenprodukt zu finden.

Die Aufgabe konnte gelöst werden mit dem neuen Verfahren gemäss Anspruch 1.

Erfindungsgemäss erfolgt die Herstellung des N-[5-(Diphenylphosphinoylmethyl)-4-(4-fluorphenyl)-6-isopropylpyrimidm-2-yl]-N-methyhnethansulfonamids der Formel (I) durch Umsetzung von [4-(4-Fluorphenyl)-6-isopropyl-2-(N-methyl-N-methylsulfonyl-amino)pyrimidin-5-yl]methanol der Formel (II) 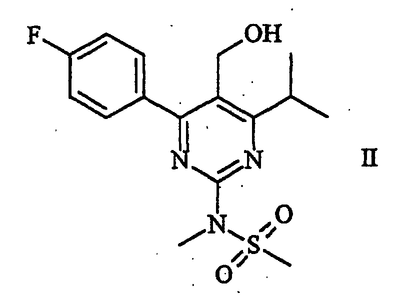 mit Chlordiphenylphosphin.

Ein bedeutender Vorteil der erfindungsgemässen Synthese ist dessen grosstechnische Verfügbarkeit.

[4-(4-Fluorphenyl)-6-isopropyl-2-(N-methyl-N-methylsulfonyl-amino)pyrimidin-5-yl]methanol der Formel (II) ist auf einfache Weise durch Reduktion des entsprechenden Esters mit z. B. Di-isobutylaluminiumhydrid zugänglich (EP-A-0521471).

Die Umsetzung von [4-(4-Fluorphenyl)-6-isopropyl-2-(N-methyl-N-methylsulfonylamino)pyrimidin-5-yl]methanol der Formel (II) mit Chlordiphenylphosphin kann entweder direkt oder über eine vorherige Deprotonierung erfolgen. Bevorzugt wird die Direktumsetzung mit Chlordiphenylphosphin mit anschliessender Behandlung mit einer Base.

Gemäss der Variante "Deprotonierung" wird das [4-(4-Fluorphenyl)-6-isopropyl-2-(Nmethyl-N-methylsulfonyl-amino)pyrimidin-5-yl] methanol der Formel (II) zweckmässig mit einem in der Fachwelt üblichen Deprotonierungsagens, vorteilhaft mit einem Alkalihydrid oder einem Alkalihexaalkyldisilazan versetzt. Bevorzugt wird Natriumhydrid eingesetzt.

Üblicherweise wird die Deprotonierung bei Raumtemperatur durchgeführt. Danach erfolgt die Umsetzung mit Chlordiphenylphosphin.

Die Umsetzung mit Chlordiphenylphosphin wird zweckmässig bei einer Temperatur zwischen 80°C und 200°C durchgeführt.

Das Chlordiphenylphosphin kann direkt als Lösungsmittel fungieren. Es ist allerdings möglich, zusätzlich ein möglichst hochsiedendes inertes wie z. B. Toluol oder Xylol oder Decalin, sowie die jeweiligen Isomerengemische, zu verwenden.

Die Umsetzung kann in Gegenwart eines Katalysators erfolgen. Als Katalysator geeignet sind Iod, Alkaliiodide wie z. B. Natrium- oder Kaliumiodid, Alkylhalogenide wie z. B. Methyl- oder Ethyliodid, oder Dihalogenalkane wie z.B. Dibromethan. Bevorzugt wird ein Alkaliiodid eingesetzt. Die Katalysatormenge wird üblicherweise im Bereich von 1 Mol% bis 20 Mol% bezogen auf den eingesetzten Alkohol der Formel II gewählt.

Die Umsetzung gemäss der direkten Variante verläuft zweckmässig bei einer Temperatur von -20°C bis 130°C, vorzugsweise bei +20°C bis 120°C.

Das Chlordiphenylphosphin kann entsprechend der Variante "Deprotonierung" direkt als Lösungsmittel fungieren. Es ist allerdings möglich, zusätzlich ein möglichst hochsiedendes inertes Lösungsmittel, wie z. B. Toluol oder Xylol oder Decalin, sowie die jeweiligen Isomerengemische, zu verwenden.

Im Anschluss an die Umsetzung mit Chlordiphenylphosphin wird das Reaktionsgemisch mit einer Base versetzt. Geeignete Basen sind Alkalihydroxide oder Alkalicarbonate, wie z.B. eine wässrige Lösung von Natrium- oder Kaliumhydroxid oder von Natrium- oder Kaliumcarbonat.

Gegebenenfalls kann zur Beschleunigung der Umsetzung mit der Base ein üblicher Phasentransferkatalysator, wie z.B. ein Tetraalkylammoniumhalogenid, eingesetzt werden. Gute Resultate lassen sich auch durch die Anwendung von Kronenethern erzielen.

Das N-[5-(Diphenylphosphinoylmethyl)-4-(4-fluorphenyl)-6-isopropylpyrümdin-2-yl]-N-methylmethansulfonamid der Formel I kann nach beendeter Reaktion auf fachmännische Weise, z.B. durch Extraktion mit einem geeigneten Lösungsmittel aus dem Reaktionsgemisch sowie durch Kristallisation aus dem Gemisch, isoliert werden.

### Beispiele

### Beispiel 1

### N-[5-(Diphenylphosphinoylmethyl)-4-(4-fluorphenyl)-6-isopropylpyrimidin-2-yl]-N-methylmethansulfonamid

1,00 g (2,83 mmol) [4-(4-Fluorphenyl)-6-isopropyl-2-(N-methyl-N-methylsulfonyl-amino)pyrimidin-5-yl]methanol wurden in 5 ml cis/trans-Decalin vorgelegt und mit 204 mg (4,68 mmol) Natriumhydrid (55% Dispersion in Mineralöl) versetzt. Nach 30 min bei Raumtemperatur wurde unter kräftiger Rührung während 6 min 680 mg (2,93 mmol) Chlordiphenylphosphin zugegeben. Man versetzte mit 52,2 mg (0,35 mmol) Natriumiodid und erhitzte während 2 h 15 min auf 184 - 186 °C. Nach Abkühlen auf Raumtemperatur wurde mit 50 ml 38 - 40%iger Natriumhydrogensulfit-Lösung und 50 ml Essigsäureethylester versetzt. Die organische Phase wurde abgetrennt und die wässrige Phase mit 50 ml Essigsäureethylester extrahiert. Die vereinten organischen Phasen wurden über Magnesiumsulfat getrocknet und im Vakuum eingeengt. Man erhielt 1,74 g Rohprodukt, das durch Chromatographie über Kieselgel (Laufmittel: n-Hexan/Essigsäureethylester 1: 2) gereinigt wurde. Man erhielt 382,4 mg (25,1 %) N-[5-(Diphenylphosphinoylmethyl)-4-(4-fluorphenyl)-6-isopropylpyrimidin-2-yl]-N-methylmethansulfonamid in Form eines farblosen Feststoffs. (Schmelzpunkt 184 - 185 °C)
¹H NMR (DMSO-d⁶, 400 MHz):
   δ = 1,11 (d, J = 6,6, Hz, 6H);
   3,28 (sept, J = 6,6 Hz, 1H);
   3,40 (s, 3H);
   3,51 (s, 3H);
   4,05 (d, J = 12,6 Hz, 2H);
   7,07 (t, J = 8,9 Hz, 2H);
   7,35 (m, 2H);
   7,42 (m, 4H);
   7,5-7,9 (m,6H).
¹³C NMR (DMSO-d⁶, 100 MHz):
   δ = 21,52 (q);
   29,12 (td);
   31,94 (d);
   33,07 (q);
   41,53 (q);
   114,50 (sd);
   115,03 (dd);
   128,54 (dd);
   130,21 (dd);
   130,84 (dd);
   131,64 (dd);
   133,41 (sd);
   134,51 (sd);
   156,54 (sd);
   162,10 (sd);
   165,86 (sd);
   176,49 (sd).

### Beispiel 2

### N-[5-(Diphenylphosphinoylmethyl)-4-(4-fluorphenyl)-6-isopropylpyrimidin-2-yl]-N-methylmethansulfonamid

282,8 mg (0,80 inmol) [4-(4-Fluorphenyl)-6-isopropyl-2-(N-methyl-N-methylsulfonylamino)pyrimidin-5-yl]methanol wurden in 4,5 ml Xylol (Isomerengemisch) vorgelegt und mit 55 mg (1,30 mmol) Natriumhydrid (55% Dispersion in Mineralöl) versetzt. Nach 35 min wurde bei Raumtemperatur unter kraftiger Rührung während 5 min 185 mg (0,84 mmol) Chlordiphenylphosphin in 1,5 ml Xylol zugegeben und anschliessend 20 h auf 135 °C erhitzt. Nach Abkühlen auf Raumtemperatur wurde mit 15 ml Wasser versetzt und mit 10 ml Essigsäureethylester extrahiert. Die organische Phase wurde abgetrennt und die wässrige Phase mit 2 x 10 ml Essigsäureethylester extrahiert. Anschliessend wurden die vereinten organischen Phasen getrocknet (MgSO₄) und im Vakuum eingeengt. Man erhielt 510 mg Rohprodukt, das durch Chromatographie über Kieselgel (Laufmittel: n-Hexan/Essigsäureethylester 1: 2, dann Essigsäureethylester) gereinigt wurde, und isolierte 230 mg (53,5 %) N-[5-(Diphenylphosphinoylmethyl)-4-(4-fluorphenyl)-6-isopropylpyrimidin-2-yl]-N-methylmethansulfonamid in Form eines farblosen Feststoffs.

### Beispiel 3

### N-[5-(Diphenylphosphinoylmethyl)-4-(4-fluorphenyl)-6-isopropylpyrimidin-2-yl]-N- methylmethansulfonamid

512 mg (1,45 mmol) [4-(4-Fluorphenyl)-6-isopropyl-2-(N-methyl-N-methylsulfonylamino)pyrimidin-5 -yl]methanol wurden in 8 ml Toluol vorgelegt und mit 96 mg (2,20 mmol) Natriumhydrid (55% Dispersion in Mineralöl) versetzt. Nach 1 h wurden bei Raumtemperatur unter kräftiger Rührung während 5 min 333,5 mg (1,44 mmol) Chlordiphenylphosphin in 2,5 ml Toluol zugegeben. Man versetzte mit 28,7 mg (0,19 mmol) Natriumiodid und erhitzte 22 h auf 108 °C. Nach 6 h wurde mit weiteren 28,7 mg (0,19 mmol) Natriumiodid versetzt. Nach Abkühlen auf Raumtemperatur wurde mit 30 ml 38 - 40%iger Natriumhydrogensulfit-Lösung versetzt und mit 50 ml Essigsäureethylester extrahiert. Die organische Phase wurde abgetrennt und die wässrige Phase mit 50 ml Essigsäureethylester extrahiert. Anschliessend wurden die vereinten organischen Phasen im Vakuum eingeengt. Man erhielt 740 mg Rohprodukt, das durch Chromatographie über Kieselgel (Laufmittel: Essigsäureethylester) gereinigt wurde, und isolierte 212,7 mg (27,3 %) N-[5-(Diphenylphosphinoylmethyl)-4-(4-fluorphenyl)-6-isopropylpyrimidin-2-yl]-N-methylrnethansulfonamid in Form eines farblosen Feststoffs.

### Beispiel 4

### N-[5-(Diphenylphosphinoylmethyl)-4-(4-fluorphenyl)-6-isopropylpyrimidin-2-yl]-N-methylmethansulfonamid

502,6 mg (1.42 mmol) [4-(4-Fluorphenyl)-6-isopropyl-2-(N-methyl-N-methylsulfonylamino)pynmidm-5-yl]methanol wurden in 8 ml Xylol (Isomerengemisch) vorgelegt und mit 96,6 mg (2,21 mmol) Natriumhydrid (55% Dispersion in Mineralöl) versetzt. Nach 1 h wurden bei Raumtemperatur unter kräftiger Rührung während 5 min 340,8 mg (1,47 mmol) Chlordiphenylphosphin in 2,5 ml Xylol zugegeben. Man versetzte mit 34,6 mg (0,23 mmol) Natriumiodid und erhitzte 19 h auf 136 °C. Nach 3 h wurde mit weiteren 25,1 mg Natriumiodid versetzt. Nach Abkühlen auf Raumtemperatur wurde mit 30 ml verdünnter Natriumhydrogensulfit-Lösung versetzt und mit 50 ml Essigsäureethylester extrahiert. Die organische Phase wurde abgetrennt und die wässrige Phase mit 50 ml Essigsäureethylester extrahiert. Anschliessend wurden die vereinten organischen Phasen im Vakuum eingeengt. Man erhielt 906 mg Rohprodukt, das durch Chromatographie über Kieselgel (Laufmittel: Essigsäureethylester) gereinigt wurde, und isolierte 315,9 mg (41,3 %) N-[5-(Diphenylphosphinoylmethyl)-4-(4-fluorphenyl)-6-isopropylpyrimidin-2-yl]-N-methylmethansulfonamid in Form eines farblosen Feststoffs.

### Beispiel 5 (direkte Umsetzung)

### N-[5-(Diphenylphosphinoylmethyl)-4-(4-fluorphenyl)-6-isopropylpyrimidin-2-yl]-N-methylmethansulfonamid

1,05 g (2,95 mmol) [4-(4-Fluorphenyl)-6-isopropyl-2-(N-methyl-N-methylsulfonyl-amino)pyrimidin-5-yl]methanol wurden in 7 g Toluol unter Eisbadkühlung vorgelegt und unter Rührung mit 820 mg (3,69 mmol) Chlordiphenylphosphin versetzt. Man erhitzte 3 h auf 108 °C. Nach Abkühlen auf Raumtemperatur wurde mit 553 mg (4,43 mmol) wässriger Kalilauge (45%ig) und 97,5 mg (0,29 mmol) Tetrabutylammoniumbromid versetzt und bei 60 °C 1 h heftig gerührt. Die Heizquelle wurde entfernt und das noch warme Reaktionsgemisch mit 20 ml Wasser versetzt. Man liess langsam auf 4 °C abkühlen und filtrierte den ausgefallenen Feststoff ab. Das Produkt wurde mit kaltem Wasser und Toluol gewaschen und im Vakuum bei 40 °C getrocknet. Man isolierte 1,04 g (64,1%; Gehalt: 97,6%) N-[(5-Diphenylphosphinoylmethyl)-4-(4-fluorphenyl)-6-isopropylpyrimidin-2-yl]-N-methylmethansulfonamid in Form eines farblosen Feststoffs.

### Beispiel 6 (direkte Umsetzung)

### N-[5-(Diphenylphosphinoylmethyl)-4-(4-fluorphenyl)-6-isopropylpyrimidin-2-yl]-N-methylmethansulfonamid

2,03 g (5,69 mmol) [4-(4-Fluorphenyl)-6-isopropyl-2-(N methyl-N-methylsulfonyl-amino)pyrimidin-5-yl]methanol wurden in 13 g Toluol vorgelegt und unter Eisbadkühlung mit 1,67 g (7,51 mmol) Chlordiphenylphosphin in 2 g Toluol versetzt. Man erhitzte 2,5 h auf 111 °C. Nach Abkühlen auf Raumtemperatur wurde mit 1,08 g (8,66 mmol) einer 45%igen Kalilauge und 175 mg (0,574 mmol) Tetrabutylammoniumchlorid versetzt und bei 60 °C 2 h heftig gerührt. Zum warmen Reaktionsgemisch wurden 30 ml Wasser gegeben. Man liess kurz bei 60 °C rühren, langsam auf 4 °C abkühlen und filtrierte den ausgefallenen Feststoff ab. Das Produkt wurde mit kaltem Wasser (10 ml) und kaltem Toluol (10 ml) gewaschen und im Vakuum bei 40 °C getrocknet. Man isolierte 2,66 g (84,1%; Gehalt: 96,6%) N-[5-(Diphenylphosphinoylmethyl)-4-(4-fluorphenyl)-6-isopmp5dpyrimidin-2-yl]-N-methylmethansulfonamid in Form eines farblosen Feststoffs.

### Beispiel 7 (direkte Umsetzung)

### N-[5-(Diphenylphosphinoylmethyl)-4-(4-fluorphenyl)-6-isopropylpyrimidin-2-yl]-N-methylmethansulfonamid

2,02 g (5,69 mmol) [4-(4-Fluorphenyl)-6-isopropyl-2-(N-methyl-N-methylsulfonyl-amino)pyrimidin-5 -yl]methanol wurden in 13,1 g Toluol vorgelegt und unter Eisbadkühlung mit 1,67 g (7,51 mmol) Chlordiphenylphosphin in 1,9 g Toluol versetzt. Man erhitzte 3 h auf 109 °C. Nach Abkühlen auf Raumtemperatur wurde mit 590 mg (8,94 mmol) festem Kaliumhydroxid und 159 mg (0,582 mmol) 18-Crown-6 versetzt und bei 60 °C 3,5 h heftig gerührt. Zum warmen Reaktionsgemisch wurden 30 ml Wasser gegeben. Man liess kurz bei 60 °C rühren, langsam auf 4 °C abkühlen und filtrierte den ausgefallenen Feststoff ab. Das Produkt wurde mit kaltem Wasser (10 ml) und kaltem Toluol (10 ml) gewaschen und im Vakuum bei 40 °C getrocknet. Man isolierte 1,82 g (59,5%; Gehalt: 95,9%) N-[5-(Diphenylphosphinoylmethyl)-4-(4-fluorphenyl)-6-isopropylpyrinudin-2-yl]-N-methylmethansulfonamid in Form eines farblosen Feststoffs.

### Beispiel 8 (direkte Umsetzung)

### N-[5-(Diphenylphosphinoylmethyl)-4-(4-fluorphenyl)-6-isopropylpyrimidin-2-yl]-N-methylmethansulfonamid

2,02 g (5,69 mmol) [4-(4-Fluorphenyl)-6-isopropyl-2-(N-methyl-N-methylsulfonyl-amino)pyrimidin-5-yl]methanol wurden in 13,1 g Toluol vorgelegt und unter Eisbadkühlung mit 1,71 g (7,69 mmol) Chlordiphenylphosphin in 1,9 g Toluol versetzt. Man erhitzte 2,5 h auf 111 °C. Nach Abkühlen auf Raumtemperatur wurde mit 1,17 g (8,78 mmol) 30%iger Natronlauge und 182 mg (0,597 mmol) Tetrabutylammoniumchlorid versetzt und bei 60 °C 3, 5 h heftig gerührt. Zum warmen Reaktionsgemisch wurden 30 ml Wasser zugegeben. Man liess kurz bei 60 °C rühren, langsam auf 4 °C abkühlen und filtrierte den ausgefallenen Feststoff ab. Das Produkt wurde mit kaltem Wasser (10 ml) und kaltem Toluol (10 ml) gewaschen und im Vakuum bei 40 °C getrocknet. Man isolierte 2,18 g (71,3%; Gehalt: 97,3%) N-[5-(Diphenylphosphinoylmethyl)-4-(4-fluorphenyl)-6-isopropylpyrimidin-2-yl]-N-methylmethansulfonamid in Form eines farblosen Feststoffs (Schmelzpunkt 184-185 °C).

### Beispiel 9 (direkte Umsetzung)

### N-[5-(Diphenylphosphinoylmethyl)-4-(4-fluorphenyl)-6-isopropylpyrimidin-2-yl]-N-methylmethansulfonamid

Eine Suspension von 9,29 g (26,3 mmol) [4-(4-Fluorphenyl)-6-isopropyl-2-(N-methyl-N-methylsulfonyl-amino)pyrimidin-5-yl]methanol in 26 g Toluol wurde unter Eisbadkühlung mit 7,64 g (34,4 mmol) Chlordiphenylphosphin versetzt. Man spülte mit wenig Toluol und erhitzte 2 h auf 111 °C. Nach Abkühlen auf Raumtemperatur wurde mit 4,94 g einer 45%igen Kalilauge und 873 mg (2,71 mmol) Tetrabutylammoniumbromid versetzt und bei 60 °C 1 h heftig gerührt. Zum warmen Reaktionsgemisch wurden 100 ml Wasser zugegeben. Man liess kurz bei 60 °C rühren, liess langsam auf 4 °C abkühlen und filtrierte den ausgefallenen Feststoff ab. Das Produkt wurde mit kaltem Wasser (30 ml) und kaltem Toluol (30 ml) gewaschen und im Vakuum bei 40 °C getrocknet. Man isolierte 11,74 g (78,4%; Gehalt 94,4%) N-[5-(Diphenylphosphinoylmethyl)-4-(4-fluorphenyl)-6-isopropylpyrimidin-2-yl]-N-methylmethansulfonamid in Form eines farblosen Feststoffs.

### Beispiel 10 (direkte Umsetzung ohne Phasentransferkatalysator)

### N-[5-(Diphenylphosphinoylmethyl)-4-(4-fluorphenyl)-6-isopropylpyrimidin-2-yl]-N-methylmethansulfonamid

2,03 g (5,69 mmol) [4-(4-Fluorphenyl)-6-isopropyl-2-(N-methyl-N-methylsulfonyl-amino)pyrimidin-5-yl]methanol wurden in 13 g Toluol vorgelegt und unter Eisbadkühlung mit 1,69 g (7,60 mmol) Chlordiphenylphosphin in 1,9 g Toluol versetzt. Man erhitzte 2,5 h auf 109 °C. Nach Abkühlen auf Raumtemperatur wurde mit 1,09 g (8,74 mmol) einer 45%igen Kalilauge versetzt und bei 60 °C 2 h 45 min heftig gerührt. Zum warmen Reaktionsgemisch wurden 30 ml Wasser gegeben. Man liess kurz bei 60 °C rühren, langsam auf 4°C abkühlen und filtrierte den ausgefallenen Feststoff ab. Das Produkt wurde mit kaltem Wasser (10 ml) und kaltem Toluol (10 ml) gewaschen und im Vakuum bei 40°C getrocknet. Man isolierte 2,44 g (76,8%; Gehalt: 99,1%) N-[5-(Diphenylphosphinoybnethyl)-4-(4-lluorphenyl)-6-isopropylpyrimidin-2-yl]-N-methylmethansulfonamid in Form eines farblosen Feststoffs.

### Beispiel 11 (direkte Umsetzung)

### N-[(5-Diphenylphosphinoylmethyl)-4-(4-fluorphenyl)-6-isopropylpyrimidin-2-yl]-N-methylmethansulfonamid

Eine Lösung von 60,08 g (0,170 mol) [4-(4-FIuorphenyl)-6-isopropyl-2-(N-methyl-N-methylsulfonyl-amino)pyrimidin-5-yl]methanol in ca. 485 ml Toluol wurde bei 60 °C mit 42,55 g (0,192 mol; Gehalt 99,7%) Chlordiphenylphosphin versetzt. Man liess 2,5 Stunden rühren und gab diese Lösung auf ein 60 °C warmes Gemisch aus 70,66 g Kalilauge (20%ig) und 5,04 g (0,017 mol) Tetrabutylammoniumchlorid und spülte mit 13 ml Toluol nach. Man liess 2 h bei 60 °C rühren und gab 215 ml Wasser und 108 ml Toluol zu. Bei ca. 80 °C wurde die wässrige Phase abgetrennt und die organische Phase noch zweimal mit jeweils 215 ml heissem Wasser extrahiert. Man entwässerte azeotrop am Wasserabscheider und liess während 2 h auf 0 °C abkühlen. Die ausgefallenen Kristalle wurden abfiltriert und mit 2 mal 160 ml Toluol gewaschen. Nach Trocknung im Vakuum bei 40 °C wurden 81,94 g (89,7%) N-[(5-Diphenylphosphinoylmethyl)4-(4-fluorphenyl)-6-isopropylpyrimidin-2-yl]-N-methylmethansulfonamid in Form eines farblosen Feststoffs erhalten [Gehalt (laut HPLC): 100%].

## Patentansprüche

1. Verfahren zur Herstellung von N-[5-(Diphenylphosphinoylmethyl)-4-(4-fluorphenyl)-6-isopropylpynimidin-2-yl]-N-methylmethansulfonamid der Formel 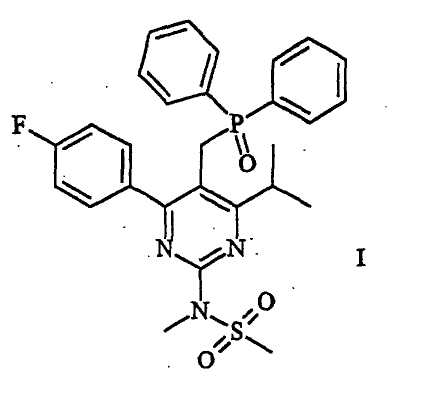 **dadurch gekennzeichnet, dass** [4-(4-Fluorphenyl)-6-isopropyl-2-(N-methyl-N-methylsulfonyl-amino)pyrimidin-5-yl]methanol der Formel 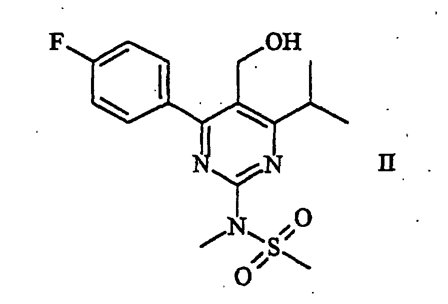 mit Chlordiphenylphosphin umgesetzt wird.

2. Verfahren nach Anspruch 1, **dadurch gekennzeichnet, dass** das [4-(4-Fluorphenyl)-6-isopropyl-2-(N-methyl-N-methylsulfonyl-amino)pyrimidin-5-yl]methanol der Formel II direkt mit Chlordiphenylphosphin und anschliessend mit einer Base umgesetzt wird.

3. Verfahren nach Anspruch 1, **dadurch gekennzeichnet, dass** das [4-(4-Fluorphenyl)-6-isopropyl-2-(N-methyl-N-methylsulfonyl-amino)pyrimidin-5-yl]methanol der Formel II zunächst deprotoniert wird.

4. Verfahren nach Anspruch 3, **dadurch gekennzeichnet, dass** die Deprotonierung mit einem Alkalihydrid oder einem Alkalihexaalkyldisilazan erfolgt.

5. Verfahren nach Anspruch 3 oder 4, **dadurch gekennzeichnet, dass** die Umsetzung mit Hilfe eines Katalysators erfolgt.

6. Verfahren nach Anspruch 5, **dadurch gekennzeichnet, dass** als Katalysator Iod, ein Alkaliiodid, ein Alkylhalogenid oder ein Dihalogenalkan eingesetzt wird.

7. Verfahren nach einem der Ansprüche 3 bis 6, **dadurch gekennzeichnet, dass** die Umsetzung mit Chlordiphenylphosphin bei einer Temperatur zwischen 80 °C und 200 °C erfolgt.

8. Verfahren nach Anspruch 2, **dadurch gekennzeichnet, dass** die direkte Umsetzung mit Chlordiphenylphosphin bei einer Temperatur zwischen -20°C und 130°C erfolgt.

9. Verfahren nach Anspruch 2 oder 8, **dadurch gekennzeichnet, dass** als Base ein Alkalihydroxid eingesetzt wird.

10. Verfahren nach Anspruch 2, 8 oder 9, **dadurch gekennzeichnet, dass** die Umsetzung mit Hilfe eines Phasentransferkatalysators erfolgt.

## Claims

1. Process for the production ofN-[5-(diphenylphosphinoylmethyl)-4-(4-fluorophenyl)-6-isopropylpyrimidin-2-yl]-N-methylmethanesulfonamide of the formula 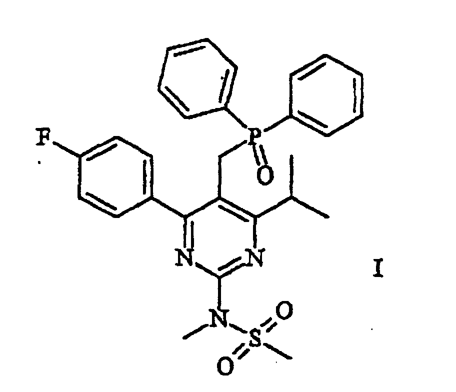 **characterised in that** [4-(4-fluorophenyl)-6-isopropyl-2-(N-methyl-N-methylsulfonylamino)pyrimidin-5-yl]methanol of the formula 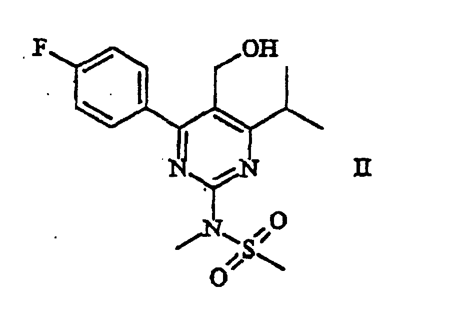 is reacted with chlorodiphenylphosphine.

2. Process according to claim 1, **characterised in that** the [4-(4-fluorophenyl)-6-isopropyl-2-(N-methyl-N-methylsulfonylamino)pyrimidin-5-yl]methanol of formula II is reacted directly with chlorodiphenylphosphine and then with a base.

3. Process according to claim 1, **characterised in that** the [4-(4-fluorophenyl)-6-isopropyl-2-(N-methyl-N-methylsulfonylamino)pyrimidin-5-yl]methanol of formula II is first deprotonated.

4. Process according to claim 3, **characterised in that** the deprotonation takes place with an alkali hydride or an alkali hexaalkyldisilazane.

5. Process according to claim 3 or 4, **characterised in that** the reaction takes place with the aid of a catalyst.

6. Process according to claim 5, **characterised in that** iodine, an alkali iodide, an alkyl halide or a dihaloalkane is used as the catalyst.

7. Process according to one of claims 3 to 6, **characterised in that** the reaction with chlorodiphenylphosphine takes place at a temperature of between 80°C and 200°C.

8. Process according to claim 2, **characterised in that** the direct reaction with chlorodiphenylphosphine takes place at a temperature of between -20°C and 130°C.

9. Process according to claim 2 or 8, **characterised in that** an alkali hydroxide is used as the base.

10. Process according to claim 2, 8 or 9, **characterised in that** the reaction takes place with the aid of a phase transfer catalyst.

## Revendications

1. Procédé de fabrication de N-[5-diphénylphosphinoylméthyl)-4-(4-fluorphényl)-6-isopropylpyrimidin-2-yl]-N-méthylméthanesulfonamide de formule 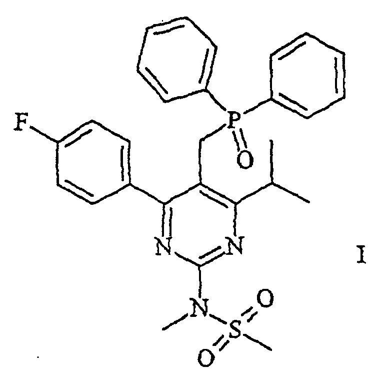 **caractérisé par le fait que** le [4-(4-fluorphényl)-6-isopropyl-2-(N-méthyl-N-méthylsulfonyl-amino)pyrimidin-5-yl]méthanol de formule 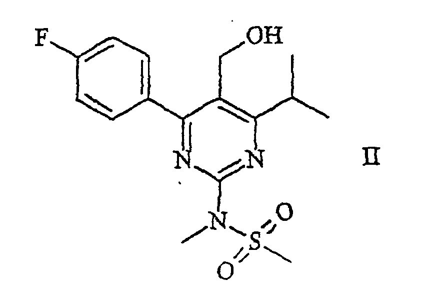 est mis à réagir avec la chlorodiphénylphosphine.

2. Procédé selon la revendication 1, **caractérisé par le fait que** le [4-(4-fluorphényl)-6-isopropyl-2-(N-méthyl-N-méthylsulfonyl -amino)pyrimidin-5-yl]méthanol de formule II est mis à réagir directement avec la chlorodiphénylphosphine et ensuite avec une base.

3. Procédé selon la revendication 1, **caractérisé par le fait que** le [4-(4-fluorphényl)-6-isopropyl-2-(N-méthyl-N-méthylsulfonyl-amino)pyrimidin-5-yl]méthanol de formule II est d'abord déprotonisé.

4. Procédé selon la revendication 3, **caractérisé par le fait que** la déprotonisation est effectuée à l'aide d'un hydrure alcalin ou d'un hexaalkyle disilazane alcalin.

5. Procédé selon la revendication 3 ou 4, **caractérisé par le fait que** la réaction est réalisée en présence d'un catalyseur.

6. Procédé selon la revendication 5, **caractérisé par le fait qu'**on utilise comme catalyseur de l'iode, un iodure alcalin, un halogénure d'alkyle ou un dihalogènoalcane.

7. Procédé selon une des revendications 3 à 6, **caractérisé par le fait que** la réaction est réalisée en présence de chlorodiphénylphosphine à une température comprise entre 80°C et 200°C.

8. Procédé selon la revendication 2, **caractérisé par le fait que** la réaction directe avec la chlorodiphénylphosphine est réalisée à une température comprise entre -20°C et 130°C.

9. Procédé selon la revendication 2 ou 8, **caractérisé par le fait que** l'on utilise un hydroxyde alcalin.

10. Procédé selon la revendication 2, 8 ou 9, **caractérisé par le fait que** la réaction est réalisée en présence d'un catalyseur de transfert de phase.
